Europäisches Patentamt

European Patent Office

Office européen des brevets

(19)

(11) Veröffentlichungsnummer : **0 451 461 B1**

(12) **EUROPÄISCHE PATENTSCHRIFT**

(45) Veröffentlichungstag der Patentschrift :
27.04.94 Patentblatt 94/17

(51) Int. Cl.⁵ : **B01F 17/34**, A61K 7/00,
A61K 47/14

(21) Anmeldenummer : 91102066.7

(22) Anmeldetag : 14.02.91

(54) **Verwendung von Mischungen aus Polyglycerinfettsäureestern als Emulgatoren in kosmetischen und pharmazeutischen Zubereitungen.**

(30) Priorität : 23.02.90 DE 4005819
25.07.90 DE 4023593

(43) Veröffentlichungstag der Anmeldung :
16.10.91 Patentblatt 91/42

(45) Bekanntmachung des Hinweises auf die
Patenterteilung :
27.04.94 Patentblatt 94/17

(84) Benannte Vertragsstaaten :
BE CH DE ES FR GB IT LI NL

(56) Entgegenhaltungen :
EP-A- 0 069 412
EP-A- 0 070 080
EP-A- 0 203 831
EP-A- 0 344 418
EP-A- 0 344 419
EP-A- 0 344 777
WO-A-81/01286
GB-A- 2 139 919
US-A- 3 936 391
US-A- 3 968 169
US-A- 4 680 184

(73) Patentinhaber : BASF Aktiengesellschaft
Carl-Bosch-Strasse 38
D-67063 Ludwigshafen (DE)

(72) Erfinder : Oppenlaender, Knut, Dr.
Otto-Dill-Strasse 23
W-6700-Ludwigshafen (DE)
Erfinder : Wegner, Brigitte, Dr.
Paul-Egell-Strasse 7
W-6720 Speyer (DE)
Erfinder : Stork, Karl, Dr.
Kurweg 65
W-6719 Carlsberg (DE)
Erfinder : Frosch, Franz, Dr.
Auf dem Koeppel 112
W-6702 Bad Duerkheim (DE)
Erfinder : Wekel, Hans-Ulrich, Dr.
Bruchstrasse 66
W-6701 Ellerstadt (DE)

**Beschreibung**

Die vorliegende Erfindung betrifft die Verwendung von Mischungen aus Polyglycerinfettsäureestern, welche durch partielle Veresterung von Polyglycerinmischungen aus

0 bis 5 Gew.-% Monoglycerin,

15 bis 40 Gew.-% Diglycerin,

30 bis 55 Gew.-% Triglycerin,

10 bis 25 Gew.-% Tetraglycerin,

0 bis 15 Gew.-% Pentaglycerin,

0 bis 10 Gew.-% Hexaglycerin und

0 bis 5 Gew.-% höheren Polyglycerinen

mit mindestens einer gesättigten Fettsäure mit 12 bis 22 C-Atomen oder mindestens einer ungesättigten Fettsäure mit 16 bis 22 C-Atomen erhältlich sind, wobei die eingesetzte ungesättigte Fettsäure oder Fettsäuremischung noch bis zu 10 Gew.-% an gesättigten Fettsäuren mit 16 bis 22 C-Atomen enthalten kann und der Veresterungsgrad der gesättigten oder ungesättigten Fettsäuren in der Mischung zwischen 20 und 70% liegt, als Emulgatoren in kosmetischen und pharmazeutischen Zubereitungen.

Weiterhin betrifft die Erfindung kosmetische und pharmazeutische Zubereitungen, die diese Mischungen als Emulgatoren enthalten.

Die EP-B 069 412 (1) betrifft ein diätetisches Getränkkonzentrat, welches neben anderen Bestandteilen einen Polyglycerinfettsäureester-Emulgator mit durchschnittlich 2 bis 10 Glycerineinheiten und 1 bis 3 Fettsäuregruppen mit 14 bis 18 C-Atomen pro Glycerineinheit enthält. Als Fettsäuren werden gesättigte Fettsäuren genannt, Stearinsäure wird nicht explizit erwähnt.

In der US-A 4 680 184 (2) werden Emulgatorzusammensetzungen für Backwaren beschrieben, die u.a. Fettsäureester von Polyolen wie Polyglycerin mit 4 bis 14 Hydroxylgruppen enthalten, wobei 10 bis 66 % der Hydroxylgruppen mit gesättigten $C_{14}$- bis $C_{20}$-Fettsäuren wie Palmitin- und Stearinsäure oder trans-ungesättigten $C_{16}$- bis $C_{20}$-Fettsäuren verestert sind.

Die EP-A 203 831 (3) betrifft Polyglycerinfettsäureester-Emulgatoren, welche überwiegend aus Mono- und Diestern von gesättigten Fettsäuren mit einer durchschnittlichen C-Atom-Zahl zwischen 13 und 16,5 bestehen. Diese Emulgatoren werden für den Nahrungsmittelsektor empfohlen.

In der Literaturstelle J. Soc. Cosmet. Chem. 28, 733-740 (Dezember 1977) (4) werden Polyglycerinester als Wasser-in-Öl-Emulgatoren für kosmetische Zubereitungen beschrieben. Als Ölsäureester werden Octaglycerinpentaoleat und Glycerintrioleat genannt.

In der Literaturstelle Fette, Seifen, Anstrichmittel, 88. Jahrgang, Nr. 3, 1986, S. 101-106 (5) werden Mono- bis Tetrafettsäureester des Diglycerins als Emulgatoren für die Lebensmittelindustrie und die Kosmetik empfohlen.

In der JP-A 80/031 038 (6), zitiert in Chem. Abstr. 93, 53797s (1980), werden Cremes und Lotionen vom Wasser-in-Öl-Typ beschrieben, die Dextrin, Diglycerinmonooleat und Diglycerindioleat enthalten.

Neben Diglycerinfettsäureestern sind auf dem Gebiet der Kosmetik außerdem Triglycerinfettsäureester und auch Triglycerinoleate verbreitet.

Die EP-B 070 080 (7) betrifft ein Verfahren zur Herstellung eines Fettes, insbesondere einer Margarinefettmasse, unter Verwendung eines Polyglycerinfettsäureesters, worin (i) der Polyglycerinanteil aus 50 bis 100% Di-, Tri- oder Tetraglycerin, 0 bis 40% Penta- oder Hexaglycerin und 0 bis 10% Heptaglycerin oder höheren Polymeren besteht, (ii) die Fettsäurereste, welche vorzugsweise gesättigt sind, aus solchen mit 18 bis 16 C-Atomen und Gemischen hiervon gewählt sind und eine Jodzahl, die 10 nicht überschreitet, besitzen und (iii) der Veresterungsgrad im Bereich von 80 bis 100% liegt.

In dem Buch "Emulgatoren für Lebensmittel", herausgegeben von G. Schuster, Springer-Verlag Berlin, Heidelberg, New York, Tokyo, 1985 (8), wird auf S. 160/161 über grenzflächenaktive Eigenschaften von Polyglycerinestern die Aussage gemacht, daß bei Veresterung aller oder der überwiegenden Anzahl der Hydroxylgruppen die Substanzen als Wasser-in-Öl-Emulgatoren, beim Vorliegen als Mono- oder Diester, also bei niedrigerem Veresterungsgrad, dagegen als Öl-in-Wasser-Emulgatoren wirken.

Die üblicherweise für kosmetische und pharmazeutische Zubereitungen verwendeten Emulgatoren sind häufig Ursache für eine noch unzureichende Lagerstabilität der Zubereitungen. So treten oftmals Ausscheidungen oder sogar mehr oder weniger vollständige Phasentrennungen bei den hergestellten Cremes auf. Weiterhin ist es wünschenswert, bei gleichbleibender Wirkung die Einsatzmenge der Emulgatoren zu verringern.

Der vorliegenden Erfindung lag die Aufgabe zugrunde, überwiegend als Wasser-in-Öl-Emulgatoren wirkende Emulgatoren für kosmetische und pharmazeutische Zubereitungen bereitzustellen, die die geschilderten Mängel nicht mehr aufweisen.

Demgemäß wurde die eingangs definierte Verwendung von Mischungen aus Polyglycerinfettsäureestern als Emulgatoren in kosmetischen und pharmazeutischen Zubereitungen gefunden.

In einer bevorzugten Ausführungsform wird eine Mischung aus Polyglycerinfettsäureestern verwendet, welche durch partielle Veresterung von Polyglycerinmischungen aus

0 bis 5 Gew.-% Monoglycerin,

20 bis 40 Gew.-% Diglycerin,

35 bis 55 Gew.-% Triglycerin,

10 bis 20 Gew.-% Tetraglycerin,

5 bis 10 Gew.-% Pentaglycerin,

1 bis 5 Gew.-% Hexaglycerin, und

0 bis 5 Gew.-% höheren Polyglycerinen

erhältlich ist.

Als gesättigte Fettsäurekomponenten eignen sich vor allem Laurinsäure, Tridecansäure, Myristinsäure, Palmitinsäure, Margarinsäure, Stearinsäure, Arachinsäure und Behensäure sowie Mischungen hieraus. Natürlich vorkommende Mischungen sind beispielsweise die Kokosfettsäuren, die als Hauptbestandteil Laurinsäure, daneben noch gesättigte $C_{14}$- bis $C_{18}$-Fettsäuren und gegebenenfalls in geringen Mengen gesättigte $C_8$- bis $C_{10}$-Fettsäuren und ungesättigte Fettsäuren enthalten, sowie Talgfettsäuren, welche im wesentlichen eine Mischung aus Palmitinsäure und Stearinsäure darstellen. Besonders bevorzugt wird eine Mischung, die aus mindestens 40 Gew.-%, vorzugsweise 60 bis 99 Gew.-% Stearinsäure besteht.

Als ungesättigte Fettsäurekomponenten eignen sich monoolefinisch ungesättigte Säuren, beispielsweise Hexadecensäuren, Octadecensäuren wie Ölsäure (cis-9-Octadecensäure) oder Elaidinsäure (trans-9-Octadecensäure), Eicosensäuren und Docosensäuren wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren wie Linolsäure und Linolensäure, sowie Mischungen hieraus. Die besten Ergebnisse erzielt man mit einer Fettsäure oder Fettsäuremischung, die aus mindestens 60 Gew.-%, vorzugsweise 65 bis 99 Gew.-% Ölsäure oder Erucasäure besteht.

Die eingesetzte ungesättigte Fettsäure oder Fettsäuremischung kann noch, bedingt durch ihre Herstellung oder Isolierung aus in der Natur vorkommenden Fettsäuregemischen, bis zu 10 Gew.-%, vorzugsweise bis zu 5 Gew.-% an gesättigten Fettsäuren mit 16 bis 22 C-Atomen enthalten, beispielsweise Palmitinsäure oder Stearinsäure.

Die Veresterung der Polyglycerine erfolgt nur partiell, der Veresterungsgrad der Mischung liegt zwischen 20 und 70%. Besonders gute Resultate erzielt man bei einem Veresterungsgrad zwischen 25 und 50%, insbesondere zwischen 30 und 40%.

Die Mischungen der Polyglycerinfettsäureester gesättigter Fettsäuren können noch bis zu 10 Gew.-%, bezogen auf die eingesetzte Menge an Fettsäuren, freie Fettsäuren oder deren Salze enthalten.

Die Mischungen der Polyglycerinfettsäureester gesättigter Fettsäuren eignen sich in kosmetischen und pharmazeutischen Zubereitungen in besonderem Maße als Öl-in-Wasser-Emulgatoren.

Die erfindungsgemäßen Emulgatoren auf Basis gesättigter Fettsäuren zeigen besonders gute Resultate, wenn sie zusammen mit bis zu 100 Gew.-%, vorzugsweise bis zu 60 Gew.-%, bezogen auf die Menge des Emulgators, eines Monoglycerinmonofettsäureesters, z.B. Glycerinmonostearat, eingesetzt werden.

Die Mischungen der Polyglycerinfettsäureester ungesättigter Fettsäuren eignen sich in kosmetischen und pharmazeutischen Zubereitungen in besonderem Maße als Wasser-in-Öl-Emulgatoren.

Die Polyglycerinfettsäureester-Mischungen stellt man zweckmäßigerweise durch Veresterungsreaktion zwischen den entsprechenden Polyglycerinmischungen und der gewünschten Fettsäure oder Fettsäuremischung nach den üblichen Methoden her. Hierbei arbeitet man in der Regel in Gegenwart eines sauren oder basischen Veresterungskatalysators wie hypophosphoriger Säure, phosphoriger Säure, Schwefelsäure, p-Toluol-sulfonsäure, Citronensäure, Natriummethylat oder einer Seife.

Die Polyglycerinmischungen sind üblicherweise gemäß der Literaturstelle (5) durch alkalisch katalysierte Kondensation von Glycerin bei erhöhten Temperaturen oder gemäß DE-A 38 42 692 (9) durch Umsetzung von Glycerin mit Epichlorhydrin in Gegenwart eines sauren Katalysators bei erhöhten Temperaturen zugänglich. Man kann aber auch entsprechende reine Polyglycerinkomponenten miteinander mischen.

Gegenstand der vorliegenden Erfindung sind weiterhin kosmetische und pharmazeutische Zubereitungen, die als Emulgatoren eine derartige Mischung aus Polyglycerinfettsäureestern in einer Menge von 0,1 bis 20 Gew.-%, vorzugsweise 0,5 bis 10 Gew.-%, insbesondere 1 bis 5 Gew.-%, bezogen auf die Gesamtmenge der Zubereitung, enthalten. Insbesondere kosmetische Zubereitungen, die diese Emulgatoren enthalten, zeigen eine deutliche Verbesserung ihrer Eigenschaften.

In Frage kommende kosmetische Zubereitungen, die durch Verwendung von Öl-in-Wasser- oder Wasser-in-Öl-Emulgatoren eine leicht streichbare Konsistenz erhalten, weil durch diese Emulgatorsysteme sich ein Öl oder ein Fett gut in eine wäßrige Phase bzw. eine wäßrige Phase gut in ein Öl oder ein Fett einarbeiten läßt,

3

sind beispielsweise Cremes wie Pflegecremes, Babycremes oder Sonnenschutzcremes, Salben, Lotionen oder Schminken. In pharmazeutischen Zubereitungen wie Salben oder Cremes benötigt man Öl-in-Wasser- oder Wasser-in-Öl-Emulgatoren zur Applikation von Wirkstoffen.

Die üblichen Zusammensetzungen und Bestandteile sowie übliche Hilfsmittel und Zusatzstoffe wie Stabilisatoren oder Konservierungsmittel für derartige kosmetische und pharmazeutische Zubereitungen sind dem Fachmann bekannt und brauchen deshalb hier nicht näher erläutert zu werden.

Die erfindungsgemäß verwendeten Polyglycerinfettsäureester-Mischungen bewirken gegenüber dem Stand der Technik eine deutlich erhöhte Lagerstabilität der mit ihnen emulgierten kosmetischen und pharmazeutischen Zubereitungen, was bei im Vergleich zu Öl-in-Wasser-Emulsionen schwerer zu stabilisierenden Wasser-in-Öl-Emulsionen besonders bedeutsam ist. Weiterhin benötigt man in der Regel von den Polyglycerinfettsäureester-Mischungen geringere Konzentrationen in den Zubereitungen, um eine gleiche Wirkung wie mit Mitteln des Standes der Technik zu erzielen.

Die Polyglycerinfettsäureester-Mischungen brauchen nach ihrer Herstellung in den meisten Fällen nicht mehr nachträglich gebleicht zu werden, da sie in der Regel bei ihrer Herstellung in genügend reiner Form anfallen. Der Verzicht auf Bleichmittel ist bei kosmetischen und pharmazeutischen Zubereitungen von großer Bedeutung, z. B. wegen der besseren Verträglichkeit solcher Zubereitungen auf der Haut oder im Körper.

Beispiel 1

Herstellung einer Polyglycerinstearat-Mischung

Unter Stickstoff als Schutzgas wurden 1417,5 g (entsprechend 7,0 mol, bezogen auf eine kryoskopisch bestimmte Durchschnittsmolmasse) einer durch Alkalimetallhydroxid katalysierte Kondensation von Glycerin erhaltenen Polyglycerin-Mischung, die gemäß gaschromatographischer Analyse aus

    35 Gew.-% Diglycerin
    39 Gew.-% Triglycerin
    18 Gew.-% Tetraglycerin
    5 Gew.-% Pentaglycerin
    2 Gew.-% Hexaglycerin
    0,6 Gew.-% Heptaglycerin
    0,3 Gew.-% Octaglycerin
    0,1 Gew.-% Nonaglycerin

bestand, zusammen mit 3335,5 g einer technischen Stearinsäure der Zusammensetzung 63 Gew.-% Stearinsäure, 28 Gew.-% Palmitinsäure, 3 Gew.-% Margarinsäure, 5 Gew.-% gesättigte Fettsäuren mit weniger als 16 C-Atomen und 1 Gew.-% gesättigte Fettsäuren mit mehr als 18 C-Atomen (entsprechend 12,0 mol, berechnet nach der gemessenen Säurezahl von 202 mg KOH/g) und 46 g 50 gew.-%iger hypophosphoriger Säure auf 180°C erhitzt und 22 Stunden bei dieser Temperatur gerührt. Während dieser Zeit wurden ca. 210 g gebildetes Reaktionswasser abdestilliert. Danach hatte das Gemisch eine Säurezahl kleiner als 10.

Nach Abkühlung auf 90 bis 95°C wurde die Reaktionsmischung mit 51 g 50 gew.-%iger Natronlauge neutralisiert. Nach einstündigem Nachrühren bei der angegebenen Temperatur und anschließender Heiß-Filtration erhielt man 4360 g einer Polyglycerinstearat-Mischung als farbloses, wachsartiges Produkt mit einer Verseifungszahl von 143, entsprechend einem Veresterungsgrad von 35 bis 40 %.

Beispiel 2

Herstellung einer Polyglycerintalgfettsäureester-Mischung

Eine Mischung aus 161,2 g (entsprechend 0,8 mol, bezogen auf eine kryoskopisch bestimmte Durchschnittsmolmasse) der in Beispiel 1 charakterisierten Polyglycerin-Mischung, 376,7 g einer gehärteten Talgfettsäure der Zusammensetzung 64 Gew.-% Stearinsäure, 27 Gew.-% Palmitinsäure, 2 Gew.-% Margarinsäure, 4 Gew.-% gesättigte Fettsäuren mit weniger als 16 C-Atomen, 2 Gew.-% gesättigte Fettsäuren mit mehr als 18 C-Atomen und 1 Gew.-% ungesättigte Fettsäuren (entsprechend 1,4 mol, berechnet nach der gemessenen Säurezahl von 208 mg KOH/g) und 2,7 g 50 gew.-%iger hypophosphoriger Säure wurde unter Stickstoff auf 180°C erhitzt. Bei dieser Temperatur wurde das gebildete Reaktionswasser innerhalb von 17 Stunden abdestilliert. Danach hatte das Gemisch eine Säurezahl kleiner als 5.

Nach Abkühlung auf ca. 90°C wurde die Reaktionsmischung mit 50 gew.-%iger Natronlauge neutralisiert. Nach Heißfiltration erhielt man 515 g einer Polyglycerintalgfettsäureester-Mischung als hellgelbes, wachsartiges Produkt mit einer Jodfarbzahl von 1 bis 3 und einem Veresterungsgrad von 35 bis 40 %.

Beispiel 3

Herstellung einer Polyglycerinoleat-Mischung

Unter Stickstoff als Schutzgas wurden 292 g (entsprechend 1,45 mol, bezogen auf eine kryoskopisch bestimmte Durchschnittsmolmasse) einer durch Alkalimetallhydroxid katalysierte Kondensation von Glycerin erhaltenen Polyglycerin-Mischung, die gemäß gaschromatographischer Analyse aus

35 Gew.-% Diglycerin
39 Gew.-% Triglycerin
18 Gew.-% Tetraglycerin
5 Gew.-% Pentaglycerin
2 Gew.-% Hexaglycerin
0,6 Gew.-% Heptaglycerin
0,3 Gew.-% Octaglycerin
0,1 Gew.-% Nonaglycerin

bestand, zusammen mit 696 g technischer Ölsäure (67 Gew.-% Ölsäure, 12 Gew.-% Linolsäure, 5 Gew.-% Hexadecensäure, 2 Gew.-% Stearinsäure, 1 Gew.-% Linolensäure, 1 Gew.-% Palmitinsäure) und 10 g hypophosphoriger Säure auf 180°C erhitzt. Bei dieser Temperatur wurde innerhalb von 12 Stunden das gebildete Reaktionswasser abdestilliert. Nach Abkühlung auf 80 bis 90°C wurde die Reaktionsmischung mit 50 gew.-%iger Natronlauge neutralisiert. Nach Filtration erhielt man 780 g einer Polyglycerinoleat-Mischung als blaßgelbe, viskose Flüssigkeit mit einer Jodfarbzahl von 3 bis 5 und einer Verseifungszahl von 145, entsprechend einem Veresterungsgrad von 35 bis 40 %.

Beispiel 4

Herstellung einer Polyglycerinerucat-Mischung

Eine Mischung aus 141 g (entsprechend 0,7 mol) der in Beispiel 1 charakterisierten Polyglycerin-Mischung, 403 g technischer Erucasäure (92 Gew.-% Erucasäure, 5 Gew.-% Eicosensäure, 2 Gew.-% Octadecensäure) und 2,7 g hypophosphoriger Säure wurde unter Stickstoff auf 180°C erhitzt. Bei dieser Temperatur wurde das gebildete Reaktionswasser innerhalb von 21 Stunden abdestilliert. Nach Abkühlung auf 80 bis 90°C wurde die Reaktionsmischung mit 50 gew.-%iger Natronlauge neutralisiert. Nach Filtration bei 80 bis 90°C erhielt man 510 g einer Polyglycerinerucat-Mischung als blaßgelben, wachsartigen Feststoff mit einer Jodfarbzahl von 4 bis 6 und einem Veresterungsgrad von 35 bis 40 %.

Vergleichsbeispiel A

Herstellung einer Polyglycerinfettsäureester-Mischung

Eine Mischung aus 194 g (entsprechend 1,0 mol, bezogen auf eine kryoskopisch bestimmte Durchschnittsmolmasse) einer durch Alkalimetallhydroxid katalysierte Kondensation von Glycerin erhaltenen Polyglycerin-Mischung, die gemäß gaschromatographischer Analyse aus

47 Gew.-% Diglycerin
25 Gew.-% Triglycerin
14 Gew.-% Tetraglycerin
8 Gew.-% Pentaglycerin
4 Gew.-% Hexaglycerin
1,3 Gew.-% Heptaglycerin
0,5 Gew.-% Octaglycerin
0,2 Gew.-% Nonaglycerin

bestand, 364 g Palmkernölfettsäure und 2,8 g p-Toluolsulfonsäure-Monohydrat wurde gemäß Beispiel 1 von (4) 3 Stunden auf 230°C erhitzt, wobei das gebildete Reaktionswasser abdestillierte. Anschließend wurde bei 100°C mit 238 g einer 50 gew.-%igen wäßrigen Citronensäure-Lösung während 2 Stunden nachverestert, die Citronensäure abgetrennt und das Rohprodukt mit 20 g einer 30 gew.-%igen wäßrigen Wasserstoffperoxid-Lösung gebleicht. Man erhielt 450 g einer Polyglycerinfettsäureester-Mischung als dunkle, viskose Flüssigkeit mit einer Jodfarbzahl von >150, einer Säurezahl von 3,3, einer Verseifungszahl von 200 und einem Veresterungsgrad von 45 bis 50 %.

Anwendungstechnische Prüfung von Polyglycerinfettsäureester-Mischungen auf Basis gesättigter Fettsäuren

Kosmetische Zubereitungen in Cremeform der folgenden Zusammensetzungen wurden auf ihre Lagerstabilität untersucht. Als Emulgatoren wurden jeweils die in der Tabelle 1 angegebenen Substanzen verwendet.

Zusammensetzung 1 (Öl-in-Wasser-Emulsion)

| Fettphase (31 Gew.-%): | 5,0 Gew.-% Emulgator |
| | 3,0 Gew.-% Glycerinmonostearat |
| | 3,0 Gew.-% Cetylalkohol |
| | 10,0 Gew.-% Cetyl-stearyl-2-ethylhexanoat |
| | 10,0 Gew.-% Paraffinöl |
| Hilfsmittel (3,5 Gew.-%): | 3,0 Gew.-% Propylenglykol |
| | 0,2 Gew.-% Methyl- und Propylparaben |
| | 0,3 Gew.-% Imidazolidinylharnstoff |
| Wasser: | 65,5 Gew.-% |

Zusammensetzung 2 (Öl-in-Wasser-Emulsion)

Wie Zusammensetzung 1, jedoch 20,0 Gew.-% Erdnußöl anstelle von 10,0 Gew.-% Cetyl-stearyl-2-ethylhexanoat und 10 Gew.-% Paraffinöl.

Zusammensetzung 3 (Öl-in-Wasser-Emulsion)

Wie Zusammensetzung 1, jedoch 20,0 Gew.-% Jojobaöl anstelle von 10,0 Gew.-% Cetyl-stearyl-2-ethylhexanoat und 10 Gew.-% Paraffinöl.

In der Tabelle 1 sind die Ergebnisse der Stabilitätsuntersuchungen wiedergegeben. Aus dem Benotungsschema erkannt man, daß die die erfindungsgemäßen Emulgatoren enthaltenden Zubereitungen bei der Lagerung bezüglich Phasenstabilität und Auftreten von Nebengeruch ausgezeichnete Ergebnisse erzielen.

Tabelle 1

Stabilität von kosmetischen Zubereitungen in Cremeform

| Emulgator | Stabilität | | | |
|---|---|---|---|---|
| | nach 6 Wochen | | nach 12 Wochen | |
| | bei 20°C | bei 45°C | bei 20°C | bei 45°C |
| Zusammensetzung 1: | | | | |
| Polyglycerinstearat-Mischung aus Bsp.1 | 1 | 1-2 | 2 | 1-2 |
| Zusammensetzung 2: | | | | |
| Polyglycerinstearat-Mischung aus Bsp.1 | 1 | 1 | 1 | 4 |
| Zusammensetzung 3: | | | | |
| Polyglycerinstearat-Mischung aus Bsp.1 | 1 | 1 | 1 | 3-4 |

Benotungsschema:
1 = stabile Emulsion, ohne Nebengeruch
2 = leicht inhomogene Emulsion, sehr schwacher Nebengeruch
3 = beginnende Ausscheidungen, schwacher Nebengeruch
4 = deutliche Ausscheidungen, starker Nebengeruch
5 = Phasentrennung

Anwendungstechnische Prüfung von Polyglycerinfettsäureester-Mischungen auf Basis ungesättigter Fettsäuren

Kosmetische Zubereitungen in Cremeform der folgenden Zusammensetzungen wurden auf ihre Lagerstabilität im Vergleich zu mit einem Mittel des Standes der Technik emulgierten Zubereitungen untersucht. Als Emulgatoren wurden jeweils die in der Tabelle 2 angegebenen Substanzen verwendet.

Zusammensetzung 4

| Fettphase (28 Gew.-%): | 1,0 Gew.-% Emulgator |
|---|---|
| | 10,0 Gew.-% Paraffinöl |
| | 10,0 Gew.-% Cetyl-stearyl-2-ethylhexanoat |
| | 3,0 Gew.-% mikrokristallines Wachs |
| | 3,0 Gew.-% Bienenwachs |
| | 0,5 Gew.-% Aluminiumstearat |

|  | |
|---|---|
| Hilfsmittel (4 Gew.-%): | 0,5 Gew.-% Magnesiumstearat |
| | 0,5 Gew.-% Magnesiumsulfat |
| | 3,0 Gew.-% Propylenglykol |
| | 0,3 Gew.-% Imidazolidinylharnstoff |
| | 0,2 Gew.-% Methyl- und Propylparaben |
| Wasser: | 68,0 Gew.-% |

Zusammensetzung 5

|  | |
|---|---|
| Fettphase (52 Gew.-%): | 5,0 Gew.-% Emulgator |
| | 10,0 Gew.-% Paraffinöl |
| | 20,0 Gew.-% Cetyl-stearyl-2-ethylhexanoat |
| | 10,0 Gew.-% hydriertes Polyisobuten |
| | 3,0 Gew.-% mikrokristallines Wachs |
| | 3,0 Gew.-% Bienenwachs |
| | 0,5 Gew.-% Aluminiumstearat |
| | 0,5 Gew.-% Magnesiumstearat |
| Hilfsmittel (4 Gew.-%): | 0,5 Gew.-% Magnesiumsulfat |
| | 3,0 Gew.-% Propylenglykol |
| | 0,3 Gew.-% Imidazolidinylharnstoff |
| | 0,2 Gew.-% Methyl- und Propylparaben |
| Wasser: | 44,0 Gew.-% |

Zusammensetzung 6

Wie Zusammensetzung 1, jedoch 3,0 Gew.-% Emulgator (Fettphase: 30 Gew.-%) und 66,0 Gew.-% Wasser.

In der Tabelle 2 sind die Ergebnisse der Stabilitätsuntersuchungen wiedergegeben. Aus dem Benotungsschema erkannt man, daß die die erfindungsgemäß verwendeten Emulgatoren enthaltenden Zubereitungen bei der Lagerung bezüglich Phasenstabilität und Auftreten von Nebengeruch deutlich bessere Ergebnisse erzielen als die Zubereitungen, welche als Emulgatoren ein Mittel des Standes der Technik enthalten.

Der Versuch, eine Polyglycerinfettsäureester-Mischung gemäß Vergleichsbeispiel A als Emulgator einzusetzen, scheiterte, weil sich sowohl hiermit behandelte Wasser-in-Paraffinöl- oder Wasser-in-Erdnußöl-Emulsionen als auch Cremezubereitungen gemäß Zusammensetzung 1 oder 2, in die diese Polyglycerinfettsäureester-Mischung eingearbeitet worden war, bereits nach spätestens 24 Stunden wieder in die einzelnen Phasen auftrennten.

Tabelle 2

Stabilität von kosmetischen Zubereitungen in Cremeform

| Emulgator | Stabilität | | | |
|---|---|---|---|---|
| | nach 6 Wochen | | nach 12 Wochen | |
| | bei 20°C | bei 45°C | bei 20°C | bei 45°C |
| Zusammensetzung 4: | | | | |
| Polyglycerinoleat-Mischung aus Bsp.3 | 1 | 1 | 1-2 | 3 |
| Mischung aus Triglycerinmono- und dioleat* zum Vergleich | 2 | 3-4 | 3 | 3-4 |
| Zusammensetzung 5: | | | | |
| Polyglycerinoleat-Mischung aus Bsp.3 | 1 | 1-2 | 1 | 2 |
| Mischung aus Triglycerinmono- und dioleat* zum Vergleich | 1 | 3 | 1-2 | 4 |
| Zusammensetzung 6: | | | | |
| Polyglycerinoleat-Mischung aus Bsp.3 | 1-2 | 1-2 | | |
| Polyglycerinerucat-Mischung aus Bsp.4 | 1-2 | 1-2 | | |

* Handelsprodukt Caprol 3 GO der Firma Capitol City Products Comp., US

Benotungsschema:
1 = stabile Emulsion, ohne Nebengeruch
2 = leicht inhomogene Emulsion, sehr schwacher Nebengeruch
3 = beginnende Ausscheidungen, schwacher Nebengeruch
4 = deutliche Ausscheidungen, starker Nebengeruch
5 = Phasentrennung

**Patentansprüche**

**Patentansprüche für folgende Vertragsstaaten : BE, CH, DE, FR, GB, IT, LI, NL**

1.  Verwendung von Mischungen aus Polyglycerinfettsäureestern, erhältlich durch partielle Veresterung von Polyglycerinmischungen aus
    0 bis 5 Gew.-% Monoglycerin,
    15 bis 40 Gew.-% Diglycerin,
    30 bis 55 Gew.-% Triglycerin,
    10 bis 25 Gew.-% Tetraglycerin,
    0 bis 15 Gew.-% Pentaglycerin,
    0 bis 10 Gew.-% Hexaglycerin und
    0 bis 5 Gew.-% höheren Polyglycerinen
    mit mindestens einer gesättigten Fettsäure mit 12 bis 22 C-Atomen oder mindestens einer ungesättigten Fettsäure mit 16 bis 22 C-Atomen, wobei die eingesetzte ungesättigte Fettsäure oder Fettsäuremischung noch bis zu 10 Gew.-% an gesättigten Fettsäuren mit 16 bis 22 C-Atomen enthalten kann und der Veresterungsgrad der gesättigten oder ungesättigten Fettsäuren in der Mischung zwischen 20 und 70 % liegt, als Emulgatoren in kosmetischen und pharmazeutischen Zubereitungen.

2.  Verwendung von Mischungen aus Polyglycerinfettsäureestern nach Anspruch 1, erhältlich durch partielle Veresterung von Polyglycerinmischungen aus
    0 bis 5 Gew.-% Monoglycerin,
    20 bis 40 Gew.-% Diglycerin,
    35 bis 55 Gew.-% Triglycerin,
    10 bis 20 Gew.-% Tetraglycerin,
    5 bis 10 Gew.-% Pentaglycerin,
    1 bis 5 Gew.-% Hexaglycerin und
    0 bis 5 Gew.-% höheren Polyglycerinen.

3.  Verwendung von Mischungen aus Polyglycerinfettsäureestern nach Anspruch 1 oder 2, erhältlich durch partielle Veresterung mit einer gesättigten Fettsäure oder Fettsäuremischung, die aus mindestens 40 Gew.-% Stearinsäure besteht.

4.  Verwendung von Mischungen aus Polyglycerinfettsäureestern nach Anspruch 1 oder 2, erhältlich durch partielle Veresterung mit einer ungesättigten Fettsäure oder Fettsäuremischung, die aus mindestens 60 Gew.-% Ölsäure oder Erucasäure besteht.

5.  Verwendung von Mischungen aus Polyglycerinfettsäureestern nach den Ansprüchen 1 bis 4, wobei der Veresterungsgrad der Polyglycerinmischungen zwischen 25 und 50 % liegt.

6.  Verwendung von Mischungen aus Polyglycerinfettsäureestern gesättigter Fettsäuren nach den Ansprüchen 1, 2, 3 oder 5 als Öl-in-Wasser-Emulgatoren in kosmetischen und pharmazeutischen Zubereitungen.

7.  Verwendung von Mischungen aus Polyglycerinfettsäureestern ungesättigter Fettsäuren nach den Ansprüchen 1, 2, 4 oder 5 als Wasser-in-Öl-Emulgatoren in kosmetischen und pharmazeutischen Zubereitungen.

8.  Kosmetische und pharmazeutische Zubereitungen, enthaltend als Emulgatoren eine Mischung aus Polyglycerinfettsäureestern gemäß den Ansprüchen 1 bis 7 in einer Menge von 0,1 bis 20 Gew.-%, bezogen auf die Gesamtmenge der Zubereitung.

**Patentansprüche für folgenden Vertragsstaat : ES**

1.  Verfahren zum Emulgieren von nicht mischbaren Phasen in kosmetischen und pharmazeutischen Zubereitungen, dadurch gekennzeichnet, daß man hierzu Mischungen aus Polyglycerinfettsäureestern verwendet, erhältlich durch partielle Veresterung von Polyglycerinmischungen aus
    0 bis 5 Gew.-% Monoglycerin,
    15 bis 40 Gew.-% Diglycerin,
    30 bis 55 Gew.-% Triglycerin,
    10 bis 25 Gew.-% Tetraglycerin,

0 bis 15 Gew.-% Pentaglycerin,
0 bis 10 Gew.-% Hexaglycerin und
0 bis 5 Gew.-% höheren Polyglycerinen
mit mindestens einer gesättigten Fettsäure mit 12 bis 22 C-Atomen oder mindestens einer ungesättigten Fettsäure mit 16 bis 22 C-Atomen, wobei die eingesetzte ungesättigte Fettsäure oder Fettsäuremischung noch bis zu 10 Gew.-% an gesättigten Fettsäuren mit 16 bis 22 C-Atomen enthalten kann und der Veresterungsgrad der gesättigten oder ungesättigten Fettsäuren in der Mischung zwischen 20 und 70 % liegt, als Emulgatoren in kosmetischen und pharmazeutischen Zubereitungen.

2. Verfahren nach Anspruch 1, dadurch gekennzeichnet, daß man von Polyglycerinmischungen aus
0 bis 5 Gew.-% Monoglycerin,
20 bis 40 Gew.-% Diglycerin,
35 bis 55 Gew.-% Triglycerin,
10 bis 20 Gew.-% Tetraglycerin,
5 bis 10 Gew.-% Pentaglycerin,
1 bis 5 Gew.-% Hexaglycerin und
0 bis 5 Gew.-% höheren Polyglycerinen
ausgeht.

3. Verfahren nach Anspruch 1 oder 2, dadurch gekennzeichnet, daß die partielle Veresterung mit einer gesättigten Fettsäure oder Fettsäuremischung, die aus mindestens 40 Gew.-% Stearinsäure besteht, erfolgt.

4. Verfahren nach Anspruch 1 oder 2, dadurch gekennzeichnet, daß die partielle Veresterung mit einer ungesättigten Fettsäure oder Fettsäuremischung, die aus mindestens 60 Gew.-% Ölsäure oder Erucasäure besteht, erfolgt.

5. Verfahren nach den Ansprüchen 1 bis 4, dadurch gekennzeichnet, daß der Veresterungsgrad der Polyglycerinmischungen zwischen 25 und 50 % liegt.

6. Verfahren nach Anspruch 1, 2, 3 oder 5, dadurch gekennzeichnet, daß man Mischungen aus Polyglycerinfettsäureestern gesättigter Fettsäuren als Öl-in-Wasser-Emulgatoren in kosmetischen und pharmazeutischen Zubereitungen einsetzt.

7. Verfahren nach Anspruch 1, 2, 4 oder 5, dadurch gekennzeichnet, daß man Mischungen aus Polyglycerinfettsäureestern ungesättigter Fettsäuren als Wasser-in-Öl-Emulgatoren in kosmetischen und pharmazeutischen Zubereitungen einsetzt.


**Claims**

**Claims for the following Contracting States : BE, CH, DE, FR, GB, IT, LI, NL**

1. Use of a mixture of polyglycerol fatty esters obtainable by partial esterification of a polyglycerol mixture consisting of
from 0 to 5% by weight of monoglycerol,
from 15 to 40% by weight of diglycerol,
from 30 to 55% by weight of triglycerol,
from 10 to 25% by weight of tetraglycerol,
from 0 to 15% by weight of pentaglycerol,
from 0 to 10% by weight of hexaglycerol and
from 0 to 5% by weight of higher polyglycerols
with one or more saturated fatty acids of 12 to 22 carbon atoms or one or more unsaturated fatty acids of 16 to 22 carbon atoms, where the unsaturated fatty acid or fatty acid mixture used may furthermore contain up to 10% by weight of saturated fatty acids of 16 to 22 carbon atoms and the degree of esterification of the saturated or unsaturated fatty acids in the mixture is from 20 to 70%, as an emulsifier in cosmetic and pharmaceutical formulations.

2. Use of a mixture of polyglycerol fatty esters as claimed in claim 1, obtainable by partial esterification of a polyglycerol mixture consisting of

from 0 to 5% by weight of monoglycerol,
from 20 to 40% by weight of diglycerol,
from 35 to 55% by weight of triglycerol,
from 10 to 20% by weight of tetraglycerol,
from 5 to 10% by weight of pentaglycerol,
from 1 to 5% by weight of hexaglycerol and
from 0 to 5% by weight of higher polyglycerols.

3. Use of a mixture of polyglycerol fatty esters as claimed in claim 1 or 2, obtainable by partial esterification with a saturated fatty acid or fatty acid mixture which consists of not less than 40% by weight of stearic acid.

4. Use of a mixture of polyglycerol fatty esters as claimed in claim 1 or 2, obtainable by partial esterification with an unsaturated fatty acid or fatty acid mixture which consists of not less than 60% by weight of oleic acid or erucic acid.

5. Use of a mixture of polyglycerol fatty esters as claimed in any of claims 1 to 4, wherein the degree of esterification of the polyglycerol mixture is from 25 to 50%.

6. Use of a mixture of polyglycerol fatty esters of saturated fatty acids as claimed in any of claims 1, 2, 3 or 5 as an oil-in-water emulsifier in cosmetic and pharmaceutical formulations.

7. Use of a mixture of polyglycerol fatty esters of unsaturated fatty acids as claimed in any of claims 1, 2, 4 or 5 as a water-in-oil emulsifier in cosmetic and pharmaceutical formulations.

8. A cosmetic or pharmaceutical formulation containing, as an emulsifier, a mixture of polyglycerol fatty esters as claimed in any of claims 1 to 7 in an amount of from 0.1 to 20% by weight, based on the total amount of the formulation.

**Claims for the following Contracting State : ES**

1. A process for emulsifying immiscible phases in cosmetic and pharmaceutical formulations, wherein a mixture of polyglycerol fatty esters is used for this purpose, said mixture being obtainable by partial esterification of a polyglycerol mixture consisting of
from 0 to 5% by weight of monoglycerol,
from 15 to 40% by weight of diglycerol,
from 30 to 55% by weight of triglycerol,
from 10 to 25% by weight of tetraglycerol,
from 0 to 15% by weight of pentaglycerol,
from 0 to 10% by weight of hexaglycerol and
from 0 to 5% by weight of higher polyglycerols
with one or more saturated fatty acids of 12 to 22 carbon atoms or one or more unsaturated fatty acids of 16 to 22 carbon atoms, where the unsaturated fatty acid or fatty acid mixture used may furthermore contain up to 10% by weight of saturated fatty acids of 16 to 22 carbon atoms and the degree of esterification of the saturated or unsaturated fatty acids in the mixture is from 20 to 70%, as an emulsifier in cosmetic and pharmaceutical formulations.

2. A process as claimed in claim 1, wherein the starting material used is a polyglycerol mixture consisting of
from 0 to 5% by weight of monoglycerol,
from 20 to 40% by weight of diglycerol,
from 35 to 55% by weight of triglycerol,
from 10 to 20% by weight of tetraglycerol,
from 5 to 10% by weight of pentaglycerol,
from 1 to 5% by weight of hexaglycerol and
from 0 to 5% by weight of higher polyglycerols.

3. A process as claimed in claim 1 or 2, wherein the partial esterification is effected with a saturated fatty acid or fatty acid mixture which consists of not less than 40% by weight of stearic acid.

4. A process as claimed in claim 1 or 2, wherein the partial esterification is effected with an unsaturated fatty acid or fatty acid mixture which consists of not less than 60% by weight of oleic acid or erucic acid.

5. A process as claimed in any of claims 1 to 4, wherein the degree of esterification of the polyglycerol mixture is from 25 to 50%.

6. A process as claimed in any of claims 1, 2, 3 or 5, wherein a mixture of polyglycerol fatty esters of saturated fatty acids is used as an oil-in-water emulsifier in cosmetic and pharmaceutical formulations.

7. A process as claimed in any of claims 1, 2, 4 or 5, wherein a mixture of polyglycerol fatty esters of unsaturated fatty acids is used as a water-in-oil emulsifier in cosmetic and pharmaceutical formulations.


**Revendications**


**Revendications pour les Etats contractants suivants : BE, CH, DE, FR, GB, IT, LI, NL**

1. Utilisation de mélanges d'esters d'acides gras de polyglycérines, que l'on peut obtenir par l'estérification partielle de mélanges de polyglycérines de
0 à 5% en poids de monoglycérine,
15 à 40% en poids de diglycérine,
30 à 55% en poids de triglycérine,
10 à 25% en poids de tétraglycérine,
0 à 15% en poids de pentaglycérine,
0 à 10% en poids d'hexaglycérine et
0 à 5% en poids de polyglycérines supérieures,
avec au moins un acide gras saturé comportant de 12 à 22 atomes de carbone, ou au moins un acide gras insaturé comportant de 16 à 22 atomes de carbone, où l'acide gras insaturé ou le mélange d'acides gras insaturés mis en oeuvre peut contenir jusqu'à encore 10% en poids d'acides gras saturés comportant de 16 à 22 atomes de carbone et le degré d'estérification des acides gras saturés ou insaturés dans le mélange fluctue entre 20 et 70%, à titre d'émulsifs dans des préparations cosmétiques et pharmaceutiques.

2. Utilisation de mélanges d'esters d'acides gras de polyglycérines suivant la revendication 1, que l'on peut obtenir par l'estérification partielle de mélanges de polyglycérines de
0 à 5% en poids de monoglycérine,
20 à 40% en poids de diglycérine,
35 à 55% en poids de triglycérine,
10 à 20% en poids de tétraglycérine,
5 à 10% en poids de pentaglycérine,
1 à 5% en poids d'hexaglycérine,
0 à 5% en poids de polyglycérines supérieures.

3. Utilisation de mélanges d'esters d'acides gras de polyglycérines suivant la revendication 1 ou 2, que l'on peut obtenir par l'estérification partielle avec un acide gras saturé ou un mélange d'acides gras saturés, qui se compose d'au moins 40% d'acide stéarique.

4. Utilisation de mélanges d'esters d'acides gras de polyglycérines suivant la revendication 1 ou 2, que l'on peut obtenir par l'estérification partielle avec un acide gras insaturé ou un mélange d'acides gras insaturés, qui se compose d'au moins 60% en poids d'acide oléique ou d'acide érucique.

5. Utilisation de mélanges d'esters d'acides de polyglycérines suivant les revendications 1 à 4, où le degré d'estérification des mélanges de polyglycérines fluctue entre 25 et 50%.

6. Utilisation de mélanges d'esters d'acides gras de polyglycérines d'acides gras saturés suivant les revendications 1, 2, 3 ou 5, à titre d'émulsifs huile-dans-eau dans des préparations cosmétiques et pharmaceutiques.

7. Utilisation de mélanges d'esters d'acides gras de polyglycérines d'acides gras insaturés suivant les revendications 1, 2, 4, ou 5, à titre d'émulsifs eau-dans-huile dans des préparations cosmétiques et pharmaceutiques.

8. Préparations cosmétiques et pharmaceutiques qui contiennent, à titre d'émulsifs, un mélange d'esters d'acides gras de polyglycérines suivant les revendica tions 1 à 7, en une proportion de 0,1 à 20% en poids, par rapport à la quantité totale de la préparation.

**Revendication pour l'Etat contractant suivant : ES**

1. Procédé d'émulsionnement de phase non miscibles dans des préparations cosmétiques et pharmaceutiques, caractérisé en ce que, à cette fin, on utilise des mélanges d'esters d'acides gras de polyglycérines, que l'on peut obtenir par l'estérification partielle de mélanges de polyglycérines de
0 à 5% en poids de monoglycérine,
15 à 40% en poids de diglycérine,
30 à 55% en poids de triglycérine,
10 à 25% en poids de tétraglycérine,
0 à 15% en poids de pentaglycérine,
0 à 10% en poids d'hexaglycérine et
0 à 5% en poids de polyglycérines supérieures,
avec au moins un acide gras saturé comportant de 12 à 22 atomes de carbone, ou au moins un acide gras insaturé comportant de 16 à 22 atomes de carbone, où le mélange d'acide gras insaturé ou le mélange d'acides gras insaturés mis en oeuvre peut contenir jusqu'à encore 10% en poids d'acides gras saturés comportant de 16 à 22 atomes de carbone et le degré d'estérification des acides gras saturés ou insaturés dans le mélange fluctue entre 20 et 70%, à titre d'émulsifs dans des préparations cosmétiques et pharmaceutiques.

2. Procédé suivant la revendication 1, caractérisé en ce que l'on part de
0 à 5% en poids de monoglycérine,
20 à 40% en poids de diglycérine,
35 à 55% en poids de triglycérine,
10 à 20% en poids de tétraglycérine,
5 à 10% en poids de pentaglycérine,
1 à 5% en poids d'hexaglycérine et
0 à 5% en poids de polyglycérines supérieures.

3. Procédé suivant la revendication 1 ou 2, caractérisé en ce que l'estérification partielle s'effectue avec un acide gras saturé ou un mélange d'acides gras saturés qui se compose d'au moins 40% en poids d'acide stéarique.

4. Procédé suivant la revendication 1 ou 2, caractérisé en ce que l'estérification partielle s'effectue avec un acide gras insaturé ou un mélange d'acides gras insaturés qui se compose d'au moins 60% en poids d'acide oléique ou d'acide érucique.

5. Procédé suivant les revendications 1 à 4, caractérisé en ce que le degré d'estérification des mélanges de polyglycérines fluctue entre 25 et 50%.

6. Procédé suivant la revendication 1, 2, 3 ou 5, caractérisé en ce que l'on utilise des mélanges d'esters d'acides gras de polyglycérines d'acides gras saturés à titre d'émulsifs huile-dans-eau dans des préparations cosmétiques et pharmaceutiques.

7. Procédé suivant la revendication 1, 2, 4 ou 5, caractérisé en ce que l'on utilise des mélanges d'esters d'acides gras de polyglycérines d'acides gras insaturés à titre d'émulsifs eau-dans-huile dans des préparations cosmétique et pharmaceutiques.